(19) Europäisches Patentamt
European Patent Office
Office européen des brevets

(11) Veröffentlichungsnummer : **0 161 418**
**B1**

(12) # EUROPÄISCHE PATENTSCHRIFT

(45) Veröffentlichungstag der Patentschrift : **07.09.88**

(51) Int. Cl.⁴ : **A 01 N 43/56**, C 07 D403/04

(21) Anmeldenummer : **85103103.9**

(22) Anmeldetag : **18.03.85**

(54) **2-(1H-Pyrazol-1-yl)-4-(3h)-chinazolinone enthaltende mikrobizide Mittel.**

(30) Priorität : **31.03.84 DE 3412080**

(43) Veröffentlichungstag der Anmeldung : **21.11.85 Patentblatt 85/47**

(45) Bekanntmachung des Hinweises auf die Patenterteilung : **07.09.88 Patentblatt 88/36**

(84) Benannte Vertragsstaaten : **BE CH DE FR GB IT LI NL SE**

(56) Entgegenhaltungen :
**DD-A- 2 001 534**
**CHEMICAL ABSTRACTS, Band 62, Nr. 9, 26. April 1965, Spalte 10451-c, Columbus, Ohio, US; & JP - A - 64 23408 (SANKYO CO.,LTD.) 20-10-1964**

(73) Patentinhaber : **BAYER AG**
**Konzernverwaltung RP Patentabteilung**
**D-5090 Leverkusen 1 Bayerwerk (DE)**

(72) Erfinder : **Gauss, Walter, Dr.**
**Ludwig-Aschoff-Strasse 4**
**D-5000 Köln 80 (DE)**
Erfinder : **Kabbe, Hans-Joachim, Dr.**
**Walter-Flex-Strasse 16**
**D-5090 Leverkusen (DE)**
Erfinder : **Paulus, Wilfried, Dr.**
**Deswatinesstrasse 90**
**D-4150 Krefeld 1 (DE)**
Erfinder : **Rosslenbroich, Hans-Jürgen, Dr.**
**Rembrandtstrasse 20**
**D-4019 Monheim (DE)**
Erfinder : **Brandes, Wilhelm, Dr.**
**Eichendorffstrasse 3**
**D-5653 Leichlingen (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 161 418 B1

Jouve, 18, rue St-Denis, 75001 Paris, France

**Beschreibung**

Die Erfindung betrifft eine neue Verwendung für 2-(1H-Pyrazol-1-yl)-4-(3H)-chinazolinone.

Aus der JA 64/23409 ist bekannt, daß die 2-(1H-Pyrazol-1-yl)-4-(3H)-chinazolinone eine Wirkung gegen Tuberkulose haben.

In der DD 20 01 53/4 ist beschrieben, die 2-(1H-Pyrazol-1-yl)-4-(3H)-chinazolinone als Thrombozyten-Aggregationshemmer sowie als Antihistaminika einzusetzen.

Es wurde nun gefunden, daß 2-(1H-Pyrazol-1-yl)-4-(3H)-chinazolinone der Formel I

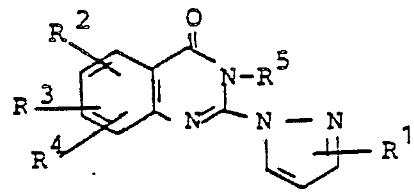

(I)

in der

$R^1$, $R^2$, $R^3$ und $R^4$ gleich oder verschieden sind und Wasserstoff, Halogen, Alkyl, Trifluormethyl, Alkoxy, Hydroxy oder Amino bedeuten und

$R^5$ für Wasserstoff oder ein Kation steht,

als Mikrobizide zum Schutz technischer Materialien und als Fungizide und Bakterizide im Pflanzenschutz ausgezeichnet geeignet sind.

In der Formel der erfindungsgemäß zu verwendenden 2-(1H-Pyrazol-1-yl)-4-(3H)-chinazolinone bedeutet Alkyl im allgemeinen geradkettiger oder verzweigter Kohlenwasserstoffrest mit 1 bis 12 Kohlenstoffatomen. Bevorzugt ist ein Niederalkylrest mit 1 bis 6 Kohlenstoffatomen. Beispielsweise seien die folgenden Alkylreste genannt : Methyl, Ethyl, Propyl, Isopropyl, Butyl, Isobutyl, Pentyl, Isopentyl, Hexyl und Isohexyl.

Alkoxy bedeutet im allgemeinen ein über Sauerstoff gebundener geradkettiger oder verzweigter Kohlenwasserstoffrest mit 1 bis 12 Kohlenstoffatomen. Bevorzugt ist ein Niederalkoxyrest mit 1 bis 6 Kohlenstoffatomen. Beispielsweise seien die folgenden Alkoxyreste genannt : Methoxy, Ethoxy, Propoxy, Isopropoxy, Butoxy, Isobutoxy, Pentoxy, Isopentoxy, Hexoxy und Isohexoxy.

Halogen bedeutet im allgemeinen Fluor, Chlor, Brom und Iod, bevorzugt Fluor, Chlor und Brom, insbesondere bevorzugt Chlor.

Kationen im Rahmen der vorliegenden Erfindung sind Alkali- und Erdalkalimetallionen, bevorzugt Natrium, Kalium, Magnesium und Kalzium, und Ammonium.

Bevorzugt werden erfindungsgemäß 2-(1H-Pyrazol-1-yl)-4-(3H)-chinazolinone der Formel II

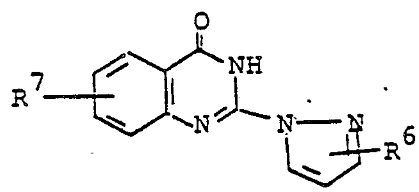

(II)

verwendet, in der $R^6$ und $R^7$ gleich oder verschieden sind und Wasserstoff, Fluor, Chlor, Brom oder $C_1$-$C_6$-Alkyl bedeuten.

Besonders bevorzugt verwendet werden erfindungsgemäß 2-(1H-Pyrazol-1-yl)-4-(3H)-chinazolinone der Formel

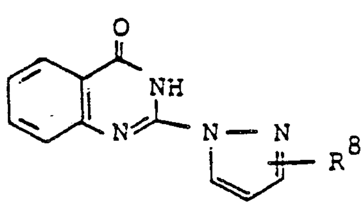

(III)

in der $R^8$ Wasserstoff oder ein Methyl-, Ethyl- oder Chlor-substituent in 3- oder 5-Stellung bedeutet.

Bevorzugt werden erfindungsgemäß Isomerengemische der 2-(1H-Pyrazol-1-yl)-4-(3H)-chinazolinone eingesetzt. Besonders bevorzugt wird ein Isomerengemisch aus 2-(3-Methylpyrazol-1-yl)-4-(3H)-chinazolinon und 2-(5-Methylpyrazol-1-yl)-4-(3H)-chinazolinon verwendet.

Die Herstellung der erfindungsgemäß zu verwendenden 2-(1H-Pyrazol-1-yl)-4-(3H)-chinazolinone

kann beispielsweise durch die folgende Reaktionsgleichung erläutert werden :

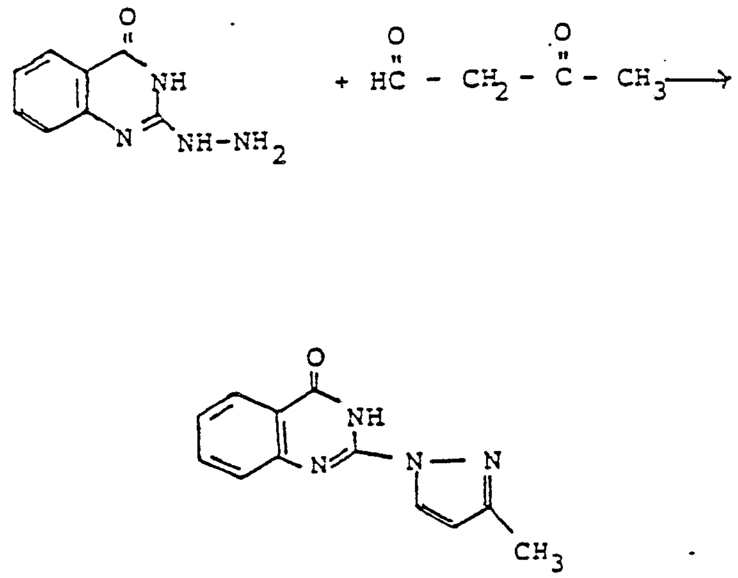

Nach diesem Verfahren setzt man ein 2-Hydrazino-4-(3H)-chinazolinon mit einer Dicarbonylverbindung oder deren Äquivalenten um : als Äquivalente der Dicarbonylverbindung können Acetale, Ketale, Enolether oder Enamine der beiden Carbonylgruppen eingesetzt werden.

Die Methode des Pyrazolringschlusses aus Hydrazin oder seinen Monosubstitutionsprodukten mit 1,3-Dicarbonylverbindungen oder Äquivalenten hierzu ist im Prinzip bekannt (Organic Syntheses 31, 43 (1951) ; BE 656.016 ; H. Plümpe und E. Schegk, Archiv der Pharmazie 300, 704 bis 708 (1967/8) ; DE-OS 2.158.490 ; DE-OS 2.802.488). Ein weiteres Verfahren zur Herstellung der erfindungsgemäß zu verwendenden 2-(1H-Pyrazol-1-yl)-4-(3H)-chinazolinone kann durch die folgende Reaktionsgleichung erläutert werden :

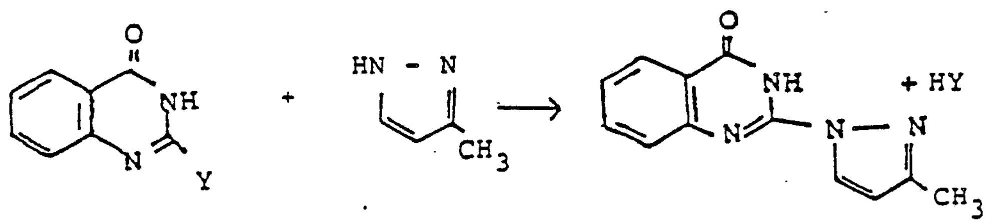

Nach diesem Verfahren kann man Chinazolinone, in denen Y für eine nucleophil austauschbare Gruppe steht, mit Pyrazolen unter Abspaltung von HY zu den erfindungsgemäß zu verwendenden Endprodukten umsetzen. Die nucleophil austauschbare Gruppe Y steht insbesondere für Halogen wie Chlor oder Brom, eine Alkylmercaptogruppe, eine Alkylsulfonylgruppe oder den Sulfonsäurerest ($SO_3H$).

Bei den erfindungsgemäß zu schützenden technischen Materialien handelt es sich um nicht lebende Materialien, die für die Verwendung in der Technik zubereitet worden sind. Beispielsweise können technische Materialien, die erfindungsgemäß vor einer mikrobiellen Veränderung oder Zerstörung geschützt werden sollen, Klebstoffe, Leime, Papier und Karton, Textilien, Leder, Holz, Anstrichmittel und Kunststoffartikel, Kühlschmiermittel und andere Materialien sein, die durch Mikroorganismen zersetzt werden können. Im Rahmen der zu schützenden Materialien seien auch Teile von Produktionsanlagen, beispielsweise Kühlwasserkreisläufe genannt, die durch Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittelund Kühlkreisläufe genannt. Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Bakterien, Schimmelpilze, insbesondere holzverfärbende und holzzerstörende Pilze (Basidiomyceten), sowie gegen Schleimorganismen und Algen.

Es seien beispielsweise Mikroorganismen der folgenden Gattungen genannt :

Alternaria, wie Alternaria tenuis,
Aspergillus, wie Aspergillus niger,
Chaetomium, wie Chaetomium globosum,
Coniophora, wie Coniophora cerebella,

Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Staphylococcus, wie Staphylococcus aureus.

Je nach Anwendungsgebiet können die erfindungsgemäß zu verwendenden Chinazolinone in die üblichen Formulierungen überführt werden, wie Lösungen, Emulsionen, Suspensionen, Pulver, Pasten und Granulate.

Diese Formulierungen können in an sich bekannter Weise hergestellt werden, z. B. durch Vermischen der Wirkstoffe mit einem Streckmittel, das aus flüssigem Lösungsmittel und/oder festen Trägerstoffen besteht, gegebenenfalls unter Verwendung von oberflächenaktiven Mitteln, wie Emulgatoren und/oder Dispergiermitteln, wobei beispielsweise im Falle der Betnutzung von Wasserstreckmitteln gegebenenfalls organische Lösungsmittel wie Alkohole als Hilfsmittel verwendet werden können.

Organische Lösungsmittel für die Wirkstoffe können beispielsweise Alkohole, wie niedere aliphatische Alkohole, vorzugsweise Ethanol oder Isopropanol, oder Benzylalkohol, Ketone wie Aceton oder Methylethylketon, flüssige Kohlenwasserstoffe, wie Benzinfraktionen, halogenierte Kohlenwasserstoffe, wie 1,2-Dichlorethan sein. Die mikrobiziden Mittel enthalten die Wirkstoffe im allgemeinen in einer Menge von 1 bis 95 %, bevorzugt von 10 bis 75 %. Die Anwendungskonzentration der erfindungsgemäß zu verwendenden Chinazolinone richtet sich nach der Art und dem Vorkommen der zu bekämpfenden Mikroorganismen, sowie nach der Zusammensetzung des zu schützenden Materials. Die optimale Einsatzmenge kann durch Testreihen ermittels werden. Im allgemeinen liegen die Anwendungskonzentrationen im Bereich von 0,001 bis 5 Gew.-%, vorzugsweise von 0,05 bis 1,0 Gew.-%, bezogen auf das zu schützende Material.

Die erfindungsgemäß zu verwendenden Chinazolinone können auch in Mischung mit anderen bekannten Wirkstoffen vorliegen. Beispielsweise seien die folgenden Wirkstoffe genannt : Benzylalkohol-mono(poly)hemiformal und andere Formaldehyd abspaltende Verbindungen, Benzimidazolyl-methylcarbamate, Tetramethyl-thiuramdisulfid, Zinksalze von Dialkylthiocarbamaten, 2,4,5,6-Tetrachlorisophthalonitril, Thiazolylbenzimidazol, Mercaptobenzthiazol, Trialkylzinnverbindungen, Methylenbisthiocyanat und Phenolderivate, wie 2-Phenylphenol, (2,2'-Dihydroxy-5,5'-dichlor)-diphenylmethan und 3-Methyl-4-chlor-phenol.

Die erfindungsgemäß zu verwendenden Chinazolinone sind auch für den Einsatz in Pflanzenschutzmitteln geeignet.

Aufgrund ihrer fungiziden Wirkung können sie im Pflanzenschutz eingesetzt werden zur Bekämpfung von Plasmodiophoromycetes, Oomycetes, Chytridiomycetes, Zygomycetes, Ascomycetes, Basidiomycetes, Deuteromycetes.

Aufgrund ihrer bakteriziden Wirkung können die erfindungsgemäß zu verwendenden Chinazolinone im Pflanzenschutz eingesetzt werden zur Bekämpfung von Pseudomonadaceae, Rhizobiaceae, Enterobacteriaceae, Corynebacteriaceae und Streptomycetaceae.

Die gute Pflanzenverträglichkeit der erfindungsgemäß zu verwendenden Chinazolinone in den zur Bekämpfung von Pflanzenkrankheiten notwendigen Konzentrationen erlaubt eine Behandlung von oberirdischen Pflanzenteilen, von Pflanzen- und Saatgut, und des Bodens.

## Beispiele

### Herstellungsbeispiele

### Beispiel 1

Ein Gemisch aus 35,2 g (0,2 Mol) 2-Hydrazino-4(3H)-chinazolinon, 250 ml Wasser, 30 ml Methanol und 36,6 ml konz. Salzsäure wird bei 40 °C mit 36,08 g (0,22 Mol) 1,1,3,3-Tetramethoxy-propan verrührt. Aus der zunächst entstehenden Lösung fällt nach kurzer Zeit das gewünschte Produkt aus. Nach insgesamt einstündiger Reaktion bei 40 °C isoliert man 29,8 g 2-(1H-Pyrazol-1-yl)-4(3H)-chinazolinon (Fp. 148-50°).

### Beispiele 2 bis 9

Aus den entsprechenden 2-Hydrazino-4(3H)-chinazolinonen und 1,1,3,3-Tetramethoxy-propan werden in analoger Weise hergestellt :

### Beispiel 2

2-(1H-Pyrazol-1-yl)-6-brom-4(3H)-chinazolinon
Fp : 210-212°

Beispiel 3

2-(1H-Pyrazol-1-yl)-5-chlor-4(3H)-chinazolinon
Fp : 195-197°

Beispiel 4

2-(1H-Pyrazol-1-yl)-6-chlor-4(3H)-chinazolinon
Fp : 217-219°

Beispiel 5

2-(1H-Pyrazol-1-yl)-7-chlor-4(3H)-chinazolinon
Fp : 221-222°

Beispiel 6

2-(1H-Pyrazol-1-yl)-6-fluor-4(3H)-chinazolinon
Fp : 196-197°

Beispiel 7

2-(1H-Pyrazol-1-yl)-8-methyl-4(3H)-chinazolinon
Fp : 166-167°

Beispiel 8

2-(1H-Pyrazol-1-yl)-6-trifluormethyl-4(3H)-chinazolinon
Fp : 220-222°

Beispiel 9

2-(1H-Pyrazol-1-yl)-6,7-dimethoxy-4(3H)-chinazolinon
Fp : 215-217°

Beispiel 10

Ein bei 40 °C gerührtes Gemisch aus 211,2 g (1,2 Mol) 2-Hydrazino-4(3H)-chinazolinon, 1,5 Ltr. Wasser, 540 ml Methanol und 220 ml konz. Salzsäure wird auf einmal mit 149,2 g (1,32 Mol) 2-Methyl-3-dimethylaminoacrolein versetzt. Die Suspension wird insgesamt 4,5 Stunden bei 40° une 1,5 Stunden bei 60° gerührt. Das bei Raumtemperatur isolierte rohe 2-(4-Methyl-1H-pyrazol-1-yl)-4(3H)-chinazolinon wird in einer Menge von 245 g mit dem Fp. 169-71° erhalten.

Beispiele 11 bis 20

Aus den entsprechenden 2-Hydrazino-4(3)-chinazolinonen und 2-Methyl-3-dimethylaminoacrolein oder 2-Ethyl-3-dimethylamino-acrolein werden in ähnlicher Weise erhalten :

Beispiel 11

2-(4-Ethyl-1H-pyrazol-1-yl)-4(3H)-chinazolinon
Fp : 126-127°

Beispiel 12

2-(4-Methyl-1H-pyrazol-1-yl)-6-brom-4(3H)-chinazolinon
Fp : 235-236°

Beispiel 13

2-(4-Ethyl-1H-pyrazol-1-yl)-5-chlor-4(3H)-chinazolinon
Fp : 132-133°

Beispiel 14

2-(4-Methyl-1H-pyrazol-1-yl)-6-chlor-4(3H)-chinazolinon
Fp : 209-210°

Beispiel 15

2-(4-Ethyl-1H-pyrazol-1-yl)-6-chlor-4(3H)-chinazolinon
Fp : 178-179°

Beispiel 16

2-(4-Methyl-1H-pyrazol-1-yl)-6-fluor-4(3H)-chinazolinon
Fp : 187-188°

Beispiel 17

2-(4-Ethyl-1H-pyrazol-1-yl)-6-fluor-4(3H)-chinazolinon
Fp : 149-151°

Beispiel 18

2-(4-Methyl-1H-pyrazol-1-yl)-8-methyl-4(3H)-chinazolinon
Fp : 169-170°

Beispiel 19

2-(4-Methyl-1H-pyrazol-1-yl)-6-trifluormethyl-4(3H)-chinazolinon
Fp : 219-220°

Beispiel 20

2-(4-Ethyl-1H-pyrazol-1-yl)-6-trifluormethyl-4(3H)-chinazolinon
Fp : 194-195°

Beispiel 21

Zu einer bei 40° gerührten Mischung aus 52,8 g (0,3 Mol) 2-Hydrazino-4(3H)-chinazolinon, 375 ml Wasser, 45 ml Methanol und 55 ml konz. Salzsäure fügt man auf einmal 57 ml (0,36 Mol) 2-Isopropoxy-3-dimethylamino-acrolein.

Nach 2-stündigem Erwärmen auf 40° gibt man 300 ml Wasser hinzu und heizt 30 Minuten lang auf 90 bis 95°. Das rohe 2-(4-Isopropoxy-1H-pyrazol-1-yl)-4(3H)-chinazolinon (53,2 g vom Fp. 165-7°) kann erforderlichenfalls aus n-Propanol umgelöst werden. Der Schmelzpunkt liegt dann bei 166 bis 168 °C.

Beispiel 22

Isomerengemisch aus 2-(3-Methyl-1H-pyrazol-1-yl)-4(3H)-chinazolinon und 2-(5-Methyl-1H-pyrazol-1-yl)-4(3H)-chinazolinon :

Zu einer bei 40° gerührten Suspension aus 228,8 g (1,3 Mol) 2-Hydrazino-4(3H)-chinazolinon, 1 625 ml Wasser, 195 ml Methanol und 238 ml konz. Salzsäure fügt man auf einmal 188,8 g (1,43 Mol) 4,4-Dimethoxy-butanon-2 und hält 1 Stunde bei 40°. Nach der Umsetzung wird das Reaktionsgemisch abgesaugt, mit Wasser gewaschen und getrocknet ; man erhält 267 g Rohprodukt vom Fp. 138 bis 141 °C. Zur Entfernung schwerlöslicher Verunreinigungen rührt man mit der 10fachen Menge Toluol bei 90°, filtriert und engt unter Vakuum ein. Es bleiben 240 g des Isomerengemisches vom Fp. 139-43° zurück. Laut Kernresonanz-Spektrum enthält es 17 % des 3-Methyl- und 83 % des 5-Methyl-Isomeren.

Beispiel 23

2-(5-Methyl-1H-pyrazol-1-yl)-4(3H)-chinazolinon :

Das gemäß Beispiel 22 erhaltene Isomerengemisch wird aus der 10fachen Menge Ethanol bis zur Schmelzpunktskonstanz umkristallisiert. Man erhält die reine 5-Methyl-Verbindung vom Fp. 153 bis 154 °C.

Beispiel 24

35,2 g (0,2 Mol) 2-Hydrazino-4(3H)-chinazolinon, 500 ml Ethanol und 31,68 g (0,24 Mol) 4,4-Dimethoxy-butanon-2 werden 4,5 Stunden unter Rückfluß gekocht. Die anfängliche Suspension hat sich

danach gelöst. Man gibt bei 50° das gleiche Volumen 2n HCl von 50° hinzu, wodurch bald ein Niederschlag entsteht. Nach insgesamt vierzigminütigem Erwärmen auf 50° wird das 2-(3-Methyl-1H-pyrazol-1-yl)-4(3H)-chinazolinon durch Absaugen isoliert. Das Rohprodukt (35,9 g vom Fp. 162 bis 165 °C) wird aus Ethanol umgelöst und schmilzt dann bei 165 bis 166 °C.

Beispiele 25 bis 28

Aus den entsprechenden 2-Hydrazino-4(3H)-chinazolinonen erhält man bei der Umsetzung mit 4,4-Dimethoxy-butanon-2 unter analogen Versuchsbedingungen die folgenden Verbindungen :

Beispiel 25

2-(3-Methyl-1H-pyrazol-1-yl)-6-brom-4(3H)-chinazolinon
Fp : 227-228°

Beispiel 26

2-(3-Methyl-1H-pyrazol-1-yl)-6-chlor-4(3H)-chinazolinon
Fp : 219-221°

Beispiel 27

2-(3-Methyl-1H-pyrazol-1-yl)-6-fluor-4(3H)-chinazolinon
Fp : 186-187°

Beispiel 28

2-(3-Methyl-1H-pyrazol-1-yl)-6-trifluormethyl-4(3H)-chinazolinon
Fp : 207-208°

Beispiel 29

96 g (0,5 Mol) 2-Methylthio-4(3H)-chinozolinon werden in 250 ml DMF mit 142 g (1 Mol) 4-(β-Methoxy-ethoxy) pyrazol 40 Stunden bei Rückflußtemperatur verrührt. Nach dem Erkalten gibt man 2,5 Liter Wasser hinzu und stellt mit Salzsäure einen $p_H$-Wert von 3 ein. Das abgeschiedene Rohprodukt (97,3 g vom Fp. 119-24°) wird aus Isopropanol umkristallisiert. Man erhält 83,8 g 2-(4-β-Methoxy-ethoxy-1H-pyrazol-1-yl)-4(3H)-chinazolinon vom Fp. 125 bis 127 °C.

Beispiele 30 bis 31

Verwendet man 4-Chlor-pyrazol oder 4-Methoxy-pyrazol anstelle des 4-(β-Methoxy-ethoxy)-pyrazols, so werden bei analoger Arbeitsweise erhalten :

Beispiel 30

2-(4-Chlor-1H-pyrazol-1-yl)-4(3H)-chinazolinon
Fp : 234-235 °C

Beispiel 31

2-(4-Methoxy-1H-pyrazol-1-yl)-4(3H)-chinazolinon
Fp : 169-170 °C

Beispiel 32

12,3 g (0,05 Mol) 2—(1H-Pyrazol-1-yl)-6-chlor-4(3H)-chinazolinon ( = Verbindung aus Beispiel 4) werden in 1 Liter Wasser mit 45 ml KOH 1 Stunde lang verrührt.

Man trennt von ungelöst gebliebenen Resten des Ausgangsmaterials ab, dampft die klare Lösung im Vakuum ein, trocknet den festen Rückstand bei 110 °C und erhält 12,2 g wasserlösliches Kaliumsalz des eingesetzten Chinazolinons.

Anwendungsbeispiele

In den Beispielen werden die folgenden Wirkstoffe durch die Buchstaben A bis G bezeichnet :

A 2-(1H-Pyrazol-1-yl)-4-(3H)-chinazolinon
B 2-(4-Methyl-1H-pyrazol-1-yl)-4(3H)-chinazolinon
C 2-(3-Methyl-1H-pyrazol-1-yl)-4(3H)-chinazolinon
D 2-(5-Methyl-1H-pyrazol-1-yl)-4(3H)-chinazolinon
E Isomerengemisch aus 2-(3- und 5-Methyl-1H-pyrazol-1-yl)-4(3H)-chinazolinon
F 2-(4-Ethyl-1H-pyrazol-1-yl)-4(3H)-chinazolinon
G 2-(4-Methyl-1H-pyrazol-1-yl)-6-chlor-4-(3H)-chinazolinon
H 2-(1H-Pyrazol-1-yl)-7-chlor-4(3H)-chinazolinon

## Beispiel 33

Zum Nachweis der Wirksamkeit gegen Pilze werden die minimalen Hemm-Konzentration (MHK) von erfindungsgemäßen Wirkstoffen bestimmt :

Ein Agar, der aus Bierwürze und Pepton hergestellt wird, wird mit erfindungsgemäßen Wirkstoffen in Konzentrationen von 0,1 mg/l bis 5 000 mg/l versetzt. Nach Erstarren des Agars erfolgt Kontamination mit Reinkulturen der in der Tabelle aufgeführten Testorganismen. Nach 2wöchiger Lagerung bei 28 °C und 60 bis 70 % relativer Luftfeuchtigkeit wird die MHK bestimmt. MHK ist die niedrigste Konzentration an Wirkstoff, bei der keinerlei Bewuchs durch die verwendete Microbenart erfolgt, sie ist in der Tabelle I angegeben.

(Siehe Tabelle I Seite 9 f.)

## Beispiel 34 (Wirkung gegen Schleimorganismen)

Erfindungsgemäße Verbindungen, gelöst in wenig Aceton, werden in Konzentrationen von jeweils 0,1 bis 100 mg/l in Allens Nährlösung (Arch. Mikrobiol. 17, 34 bis 53 (1952)), die in 4 l sterilem Wasser 0,2 g Ammoniumchlorid, 4,0 g Natriumnitrat, 1,0 g Dikaliumhydrogenphosphat, 0,2 g Magnesiumsulfat, 0,02 g Eisenchlorid und 1 % Caprolactam enthält, zur Anwendung gebracht. Kurz vorher werden die Nährlösungen mit Schleimorganismen (ca. $10^6$ Keime/ml), die aus der Polyamid-Herstellung verwendeten Spinnwasser-Kreisläufen isoliert wurden, infiziert. Nährlösungen, die die minimale Hemmkonzentration (MHK) oder größere Wirkstoffkonzentrationen aufweisen, sind nach 3wöchiger Kultur bei Raumtemperatur noch völlig klar, d. h. die in wirkstoffreien Nährlösungen nach 3 bis 4 Tagen bemerkbare starke Vermehrung der Mikroben und Schleimbildung unterbleibt.

Für erfindungsgemäße Substanzen lassen sich auf diese Weise folgende MHK ermitteln :

| Wirkstoff | MHK in mg/l |
|---|---|
| A | 30 |
| C | 100 |
| D | 75 |
| F | 30 |
| H | 100 |

## Beispiel 35

Prüfung des Wirkstoffs E als Anstrichfungizid

Die Prüfung wird in Anlehnung an den Report 219 der Defense Standards Laboratories Maribyrnong/Australien wie folgt durchgeführt : Ein glatter Karton wird beidseitig mit dem zu prüfenden Anstrichmittel bestrichen und 8 Tage bei Raumtemperatur getrocknet. Zur Alterung wird ein Teil des Anstriches 24 Stunden in fließendem Wasser von 24° gewässert oder 8 Tage mit Frischluft von 40 bis 60 °C belüftet oder 110 Stunden einem trockenen Xenon-Test ausgesetzt. Von den so vorbereiteten Proben werden Abschnitte von 5 × 5 cm einzeln in Petrischalen auf einen Traubenzucker-Nährboden aufgelegt und mit einer Sporensuspension der nachstehenden Pilze kontaminiert : Aspergillus niger, Aureobasidium pullulans, Alternaria tenuis, Penicillium citrinum, Stachybotrys atra, Paecilomyces varioti, Cladosporium herbarum, Aspergillus ustus und Aspergillus flavus.

Die kontaminierten Schalen werden bei 28 bis 30 °C und 90 bis 95 % relativer Luftfeuchtigkeit gelagert und nach 3 Wochen ausgewertet. Anstriche gelten als schimmelfest, wenn die Proben nach diesen Tests pilzfrei bleiben.

Nach der oben angegebenen Testmethode wird eine handelsübliche Dispersionsfarbe auf PVA-Basis auf Schimmelfestigkeit geprüft.

Nach dem Test ergibt sich die folgende Beurteilung :

Anstriche der PVA-Dispersionsfarbe, die 0,5 % der erfindungsgemäßen Substanz enthalten, sind schimmelfest, auch wenn sie vor der mikrobiologischen Prüfung den obengenannten Alterungsprozessen

Tabelle I : Angabe der MKH-Werte in mg/l bei der Einwirkung erfindungsgemäßer Substanzen auf Pilze

| Wirkstoff | Aspergillus niger | Alternaria tenuis | Aureobasidium pullulans | Chaetomium globosum | Coniophora puteana | Lentinus tigrinus | Penicillium glaucum | Polyporus versicolor | Sclerophoma pityophila | Trichoderma viride |
|---|---|---|---|---|---|---|---|---|---|---|
| A | 100 | 20 | 5 | 100 | 1 | 20 | 100 | 20 | 20 | 100 |
| B | 50 | 75 | 10 | <20 | | <10 | 7 | <10 | 50 | 50 |
| C | 35 | 15 | 50 | 5 | <0,1 | 20 | 75 | 35 | 10 | 50 |
| D | 35 | 15 | 35 | 5 | <0,1 | 20 | 35 | 15 | 50 | 50 |
| E | 50 | 75 | 15 | 3 | 1 | 20 | 35 | 35 | 2 | 75 |
| F | 50 | | 50 | <20 | | 20 | 35 | 30 | 50 | 50 |
| G | | | | 1 | 1 | | 7 | | 5 | |

ausgesetzt wurden.

Hingegen sind Anstriche der PVA-Dispersionsfarbe, die als Fungizid Tetramethylthiuramdisulfid enthalten, erst schimmelfest, wenn sie 4 % von diesem Fungizid enthalten.

Beispiel 36 (mit Vergleich)

Prüfung des Wirkstoffs E als Konservierungsstoff für Kühlschmiermittel

Ein Kühlschmiermittel auf Mineralölbasis wird mit 3 % eines Konservierungsstoffes versetzt, der sich aus 90 % Benzylalkoholmono(poly) hemiformal und 10 % Wirkstoff E zusammensetzt.

3-5 %ige Gebrauchsverdünnungen des Kühlschmiermittels werden während 3 Monaten täglich massiv mit Mikroben (Bakterien, Hefen, Schimmelpilzen), isoliert aus mikrobiell verdorbenen Kühlschmiermittelgebrauchsverdünnungen, kontaminiert.

Die Gebrauchsverdünnungen sind am Ende der Prüfzeit noch keimfrei ; sie sind also zuverlässig konserviert.

Hingegen weisen entsprechende Gebrauchsverdünnungen, die nur Benzylalkoholmono(poly) hemiformal enthalten, am Ende der Prüfzeit Schimmelpilzbefall auf ; demnach ist die Konservierung nicht ausreichend.

**Patentansprüche**

1. Verwendung von 2-(1H-Pyrazol-1-yl)-4-(3H)-chinazolinonen der Formel

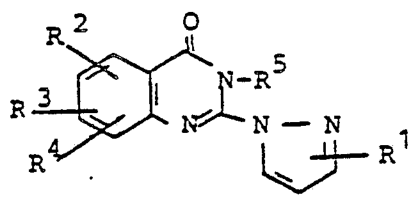

in der

$R^1$, $R^2$, $R^3$ und $R^4$ gleich oder verschieden sind und Wasserstoff, Halogen, Alkyl, Trifluormethyl, Alkoxy, Hydroxy oder Amino bedeuten und

$R^5$ für Wasserstoff oder ein Kation steht,

als Mikrobizide zum Schutz technischer Materialien und als Fungizide und Bakterizide im Pflanzenschutz.

2. Verwendung gemäß Anspruch 1 von 2-(1H-Pyrazol-1-yl)-4-(3H-)chinazolinonen der Formel

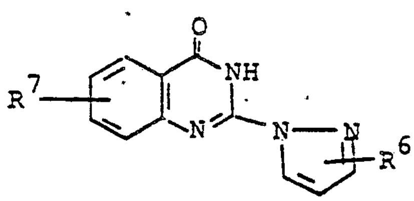

(II)

in der $R^6$ und $R^7$ gleich oder verschieden sind und Wasserstoff, Fluor, Chlor, Brom oder $C_1$-$C_6$-Alkyl bedeuten.

3. Verwendung gemäß Anspruch 1 von 2-(1H-Pyrazol-1-yl)-4-(3H)-chinazolinonen der Formel

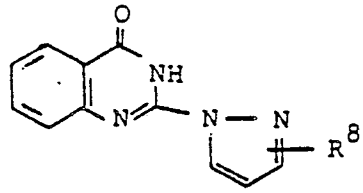

(III)

in der $R^8$ Wasserstoff oder ein Methyl-, Ethyl- oder Chlor-substituent in 3- oder 5-Stellung bedeutet.

4. Verwendung gemäß Ansprüchen 1, 2 oder 3, dadurch gekennzeichnet, daß man die 2-(1H-Pyrazol-1-yl)-4-(3H)-chinazolinone bei ihrer Verwendung als Mikrobizide in einer Menge von 0,001 bis 5 Gew.-% bezogen auf das Gewicht des zu schützenden Materials einsetzt.

**Claims**

1. Use of 2-(1H-pyrazol-1-yl)-4-(3H)-quinazolinones of the formula

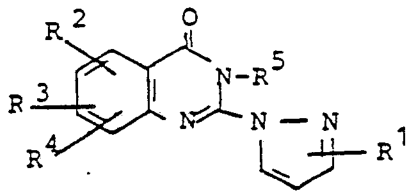

in which

$R^1$, $R^2$, $R^3$ and $R^4$ are identical or different and denote hydrogen, halogen, alkyl, trifluoromethyl, alkoxy, hydroxyl or amino, and

$R^5$ represents hydrogen or a cation,

as microbicides for protecting industrial materials, and as fungicides and bactericides in plant protection.

2. Use according to Claim 1 of 2-(1H-pyrazol-1-yl)-4-(3H)-quinazolinones of the formula

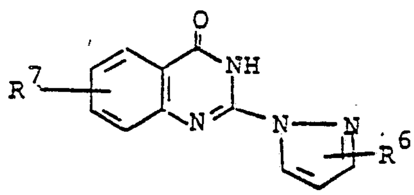

in which $R^6$ and $R^7$ are identical or different and denote hydrogen, fluorine, chlorine, bromine or $C_1$-$C_6$-alkyl.

3. Use according to Claim 1 of 2-(1H-pyrazol-1-yl)-4-(3H)-quinazolinones of the formula

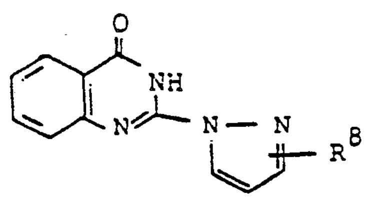

in which $R^8$ denotes hydrogen or a methyl, ethyl or chlorine substituent in the 3- or 5-position.

4. Use according to Claims 1, 2 or 3, characterized in that the 2-(1H-pyrazol-1-yl)-4-(3H)-quinazolinones are, when used as microbicides, employed in an amount of 0.001 to 5 % by weight based on the weight of the material to be protected.

**Revendications**

1. Utilisation des 2-(1H-pyrazole-1-yl)-4(3H)-quinazolinones de formule

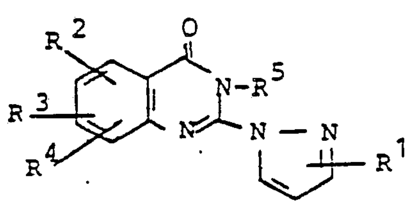

dans laquelle

$R^1$, $R^2$, $R^3$ et $R^4$, ayant des significations identiques ou différentes, représentent chacun l'hydrogène, un halogène, un groupe alkyle, trifluorométhyle, alcoxy, hydroxy ou amino, et

$R^5$ représente l'hydrogène ou un cation,

en tant que microbicides pour la protection des matériaux techniques et en tant que fongicides et bactéricides pour la protection des végétaux.

2. Utilisation selon la revendication 1 des 2-(1H-pyrazole-1-yl)-4(3H)-quinazolinones de formule

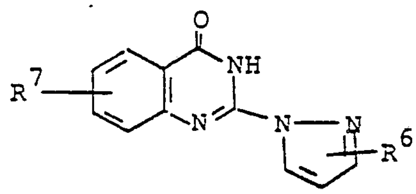

(II)

dans laquelle $R^6$ et $R^7$, ayant des significations identiques ou différentes, représentent chacun l'hydrogène, le fluor, le chlore, le brome ou un groupe alkyle en $C_1$-$C_6$.

3. Utilisation selon la revendication 1 des 2-(1H-pyrazole-1-yl)-4(3H)-quinazolinones de formule

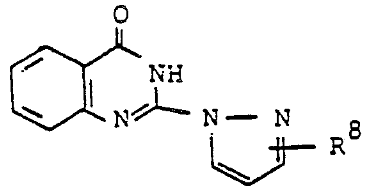

(III)

dans laquelle $R^8$ représente l'hydrogène ou un substituant méthyle, éthyle ou chloro en position 3 ou 5.

4. Utilisation selon les revendications 1, 2 ou 3, caractérisée en ce que, lorsque les 2-(1H-pyrazole-1-yl)-4(3H)-quinazolinones sont utilisées en tant que microbicides, on les utilise en quantités de 0,001 à 5 % par rapport au poids du matériau à protéger.